(19) Europäisches Patentamt
European Patent Office
Office européen des brevets

(11) EP 0 845 463 A1

(12) **EUROPEAN PATENT APPLICATION**
published in accordance with Art. 158(3) EPC

(43) Date of publication:
**03.06.1998 Bulletin 1998/23**

(21) Application number: **95924522.6**

(22) Date of filing: **07.07.1995**

(51) Int. Cl.[6]: **C07D 491/113**, A61K 31/435

(86) International application number:
**PCT/JP95/01361**

(87) International publication number:
**WO 97/03075 (30.01.1997 Gazette 1997/06)**

(84) Designated Contracting States:
**AT BE CH DE DK ES FR GB GR IE IT LI LU MC NL PT SE**

(71) Applicant:
**TAISHO PHARMACEUTICAL CO. LTD**
**Tokyo 171 (JP)**

(72) Inventors:
- **SATO, Masakazu**
**Toshima-ku, Tokyo 171 (JP)**
- **MANAKA, Akira**
**Toshima-ku, Tokyo 171 (JP)**
- **TAKAHASHI, Keiko**
**Toshima-ku, Tokyo 171 (JP)**
- **KAWASHIMA, Yutaka**
**Toshima-ku, Tokyo 171 (JP)**
- **HATAYAMA, Katsuo**
**Toshima-ku, Tokyo 171 (JP)**

(74) Representative:
**Weisert, Annekäte, Dipl.-Ing. Dr.-Ing.**
**Patentanwälte**
**Kraus Weisert & Partner**
**Thomas-Wimmer-Ring 15**
**80539 München (DE)**

(54) **THIAZOLINE DERIVATIVE**

(57) Object: To provide the thiazoline derivatives having an excellent inhibitory action of blood platelet aggregation when administered not only parenterally but also orally.

Constitution: A thiazoline derivative represented by the formula:

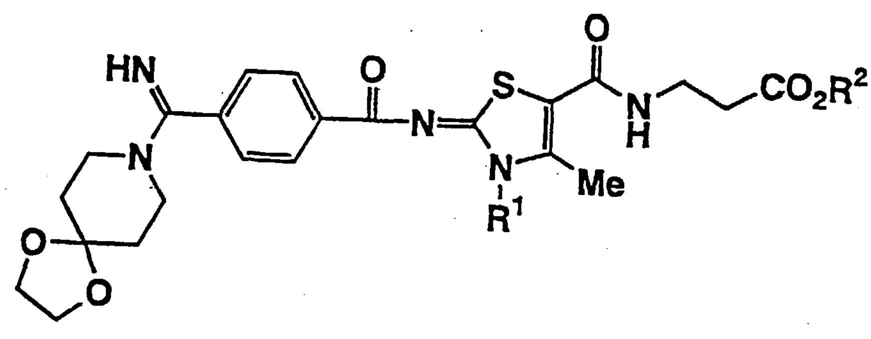

(wherein $R^1$ is an alkyl group having 1 to 4 carbon atoms, and $R^2$ is a hydrogen atom or an alkyl group having 1 to 4 carbon atoms) or a pharmaceutically acceptable salt thereof.

EP 0 845 463 A1

**Description**

TECHNICAL FIELD

The present invention relates to novel thiazoline derivatives having an excellent inhibitory action of blood platelet aggregation.

BACKGROUND ART

The compounds of the present invention are included in the scope of the general formula (I) described in the claim of WO94/02472, but not specifically described in this patent specification. The compounds disclosed specifically in the specification of WO94/02472 have an excellent inhibitory action of blood platelet aggregation by parenteral administration (e.g. by injection administration), but have such a drawback as low pharmacological activity by oral administration.

An object of the present invention is to provide compounds having an excellent inhibitory action of blood platelet aggregation even when administered orally.

DISCLOSURE OF THE INVENTION

As a result of extensive researches, the present inventors have found that certain thiazoline derivatives have a more excellent activity by oral administration than the compounds disclosed specifically in the specification of WO94/02472 and achieve the above-mentioned object, and thus the present invention has been accomplished.

The present invention is a thiazoline derivative represented by the formula:

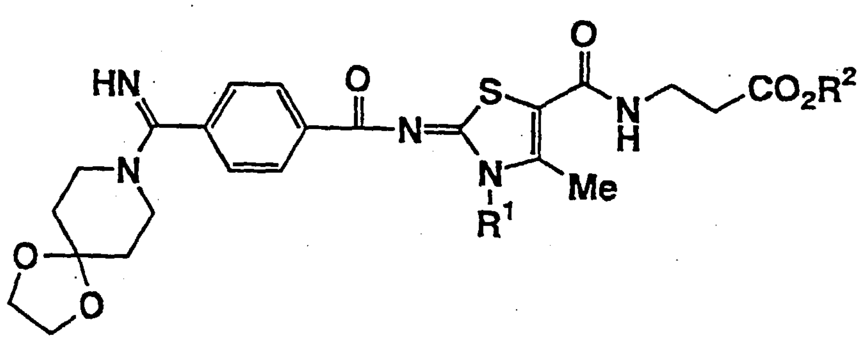

(I)

(wherein $R^1$ is an alkyl group having 1 to 4 carbon atoms, and $R^2$ is a hydrogen atom or an alkyl group having 1 to 4 carbon atoms) or a pharmaceutically acceptable salt thereof.

In the present invention, the alkyl group having 1 to 4 carbon atoms refers to a methyl group, an ethyl group, an n-propyl group, an isopropyl group, an n-butyl group, an isobutyl group, a sec-butyl group or a tert-butyl group. The pharmaceutically acceptable salt of the compound of Formula (I) refers to any pharmacologically acceptable salts, for example, salts with an alkali metal, an alkali earth metal, ammonia, an alkylamine, a mineral acid, a carboxylic acid or a sulfonic acid, and more especially sodium salt, potassium salt, calcium salt, ammonium salt, aluminum salt, triethylammonium salt, 1-adamantylamine salt, hydrochloride, hydrobromide, hydroiodide, sulfate, nitrate, phosphate, monomethylsulfate, acetate, propionate, butyrate, succinate, tartrate, citrate, tannate, malate, caproate, valerate, fumarate, maleate, methanesulfonate or tosylate, but are not limited thereto.

The compounds of the present invention can be prepared, for example, by the following methods.

That is, a compound represented by the formula:

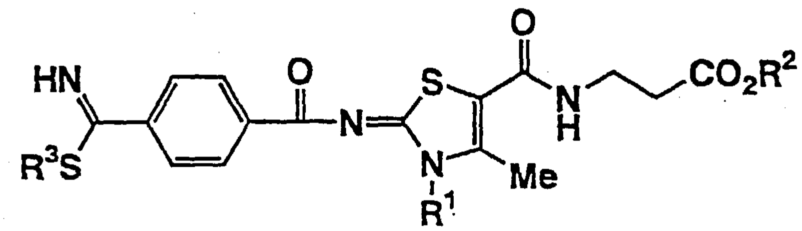

(wherein $R^3$ is a lower alkyl group, and $R^1$ and $R^2$ are as defined above) or a salt thereof, which can be prepared by the method described on page 4 of the specification of WO94/02472, is reacted with 4,4-ethylenedioxypiperidine or a salt thereof to give a compound of Formula (I) or a pharmaceutically acceptable salt thereof.

The compound of Formula (I) wherein $R^2$ is a hydrogen atom or a pharmaceutically acceptable salt thereof can be also prepared by hydrolysis of the ester moiety of the compound of Formula (I) wherein $R^2$ is an alkyl group having 1 to 4 carbon atoms or a pharmaceutically acceptable salt thereof. The hydrolysis to be used may be an ordinary method such as treatments with a base, a mineral acid or an organic acid.

The compound of Formula (I) wherein $R^2$ is an alkyl group having 1 to 4 carbon atoms can be interchanged by transesterification, for example, using an acid as a catalyst.

Examples of the base to be used in the above-mentioned reaction are an alkali metal salt (e.g. sodium carbonate, potassium carbonate, sodium bicarbonate, potassium bicarbonate, sodium hydroxide, potassium hydroxide, dimsyl sodium, sodium hydride, sodium amide or tert-butyl potassium), an amine (e.g. triethylamine, diisopropylethylamine or pyridine), sodium acetate and potassium acetate; examples of the mineral acid are hydrochloric acid, hydrobromic acid, hydroiodic acid, nitric acid and sulfuric acid; and examples of the organic acid are acetic acid, methanesulfonic acid and p-toluenesulfonic acid. Examples of the salt of 4,4-ethylenedioxypiperidine are organic acid salts such as acetate, and mineral acid salts such as hydrochloride. Examples of the reaction solvent to be used are reaction-inert solvents such as water, an alcohol (e.g. methanol, ethanol, isopropyl alcohol or tert-butyl alcohol), an ether (e.g. dioxane or tetrahydrofuran), dimethylformamide, dimethyl sulfoxide, pyridine, methylene chloride, chloroform, acetone and acetic acid.

INDUSTRIAL APPLICABILITY

The thus-obtained compounds of the present invention have an excellent inhibitory action of blood platelet aggregation when administered not only parenterally but also orally.

Accordingly, the compounds of the present invention are useful as preventive or therapeutic agents of ischemic diseases (e.g. thrombosis, cerebral infarction or myocardial infarction) and arteriosclerosis diseases.

For the purposes, the compounds of the present invention can be mixed with, for example, conventional fillers, binders, disintegrators, pH modulators or solubilizers, and prepared in the form of tablets, piles, capsules, granules, powders, solutions, emulsions, suspensions or injections by conventional formulation techniques.

The dose of the compound of the present invention for adult patients is 1 to 1000 mg per day, which can be given in several divided doses. This dose can be increased or decreased depending on the kind of the diseases and the age, body weight and condition of the patient.

The excellent action of the compound of the present invention is illustrated by the following experiment.

Experiment [Inhibitory Action Test of Guinea-pig Blood Platelet Aggregation by Oral Administration]

Groups of 4 male Hartley strain guinea-pigs, 4 weeks old, each weighing 250 to 300 g, were used for the test after an overnight fast. A suspension of the test drug in 10% Tween 80 solution was administered orally in the amount of 1 mg/kg. To control group was administered the solvent only. At 30 minutes after the oral administration, animals were anesthetized with pentobarbital (30 mg/kg, ip) and performed a laparotomy, and the blood (the volume ratio of 3.8% sodium citrate solution and blood is 1:9) was collected from the abdominal aorta using a plastic syringe. The blood was centrifuged at 120 x g for 10 minutes to give platelet rich plasma (PRP) as a supernatant. The remaining blood was further centrifuged at 1100 x g for 10 minutes to give platelet poor plasma (PPP). The platelet count of PRP was adjusted to 4 - 6 x $10^5/mm^3$ by diluting with PPP.

Blood platelet aggregation was monitored according to the method of Born G.V.R., [Nature, vol. 194, page 927 (1962)] using adenosine diphosphate (produced by Sigma Co.; hereinafter referred to as "ADP") as an aggregation-inducing substance. That is, a solution of the compound of Formula (I) as a test drug in dimethyl sulfoxide was adjusted to the desired concentration with a physiological saline solution. 25 μl of this solution was added to 250 μl of PRP and

incubated at 37°C for 3 minutes, and 25 μl of ADP (final concentration: 3 μM)/epinephrine (final concentration: 10 μM) as an aggregation-inducing agent was added thereto. The mixture was monitored for 5 minutes by using a blood platelet aggregation ability measurement apparatus (Aggricoda PAM-8C; manufactured by Mebanix Co.). From the following formula was calculated the aggregation inhibition rate of the group treated with the test drug to the maximum aggregation rate of the group treated with the solvent as a control group, and the concentration for 50% inhibition ($IC_{50}$ value) was calculated.

$$\text{Aggregation inhibition rate (\%)} = \left(1 - \frac{\text{Maximum aggregation rate of group treated with test drug}}{\text{Maximum aggregation rate of control group}}\right) \times 100$$

In addition, N-(2-carboxyethyl)-2-(4-amidinobenzoylimino)-3-isopropyl-4-methyl-3H-thiazoline-5-carboxamide hydrobromide (described in the specification of WO 94/02472) was used as a comparative drug and tested in the same manner as described above. Results are shown in Table 1.

Table 1

| Test drug | Inhibition rate ± SD (%) |
|---|---|
| Compound 3 | 99.3 ± 0.7 |
| Comparative drug | 15.0 ± 5.7 |

BEST MODE FOR CARRYING OUT THE INVENTION

The present invention is illustrated in more detail by the following examples.

Reference Example

N-(2-Methoxycarbonylethyl)-2-[4-(methylthioimidoyl)benzoylimino]-3,4-dimethyl-3H-thiazoline-5-carboxamide methylsulfate (Compound 1)

A mixture of N-(2-methoxycarbonylethyl)-2-(4-thiocarbamoylbenzoylimino)-3,4-dimethyl-3H-thiazoline-5-carboxamide (104 g), dimethyl sulfate (86.1 ml) and N,N-dimethylformamide (400 ml) was stirred at room temperature for 4 hours. To the reaction mixture was added acetone (1.5 L) under ice-cooling, and the precipitated crystals were collected by filtration and washed with acetone to give the title compound (126.5 g).
m.p. 164 - 167°C

Example 1

N-(2-Methoxycarbonylethyl)-2-{4-[(4,4-ethylenedioxypiperidin-1-yl)imidoyl]benzoylimino}-3,4-dimethyl-3H-thiazoline-5-carboxamide methylsulfate (Compound 2)

A mixture of Compound 1 (10.0 g), 4,4-ethylenedioxypiperidine (3.93 g), acetic acid (1.57 ml) and acetone (100 ml) was stirred at 80°C for 90 minutes. The reaction mixture was allowed to stand for cooling, and the precipitated crystals were collected by filtration and washed with a mixture of methanol and toluene to give the title compound (7.66 g).
m.p. 215 - 216°C (decomposed).

Example 2

N-(2-Carboxyethyl)-2-{4-[(4,4-ethylenedioxypiperidin-1-yl)imidoyl]benzoylimino}-3,4-dimethyl-3H-thiazoline-5-carboxamide trihydrate (Compound 3)

A mixture of Compound 2 (7.0 g), 5% aqueous sodium hydroxide solution (19.2 ml) and 2-propanol (70 ml) was stirred at room temperature for an hour. To the reaction mixture was added 1% aqueous phosphoric acid solution (60 ml), and the precipitated crystals were collected by filtration to give the title compound (5.3 g).
m.p. 233 - 234°C (decomposed).

Example 3

N-(2-Methoxycarbonylethyl)-2-{4-[(4,4-ethylenedioxypiperidin-1-yl)imidoyl]benzoylimino}-3,4-dimethyl-3H-thiazoline-5-carboxamide hydroiodide (Compound 4)

A mixture of N-(2-methoxycarbonylethyl)-2-[4-(methylthioimidoyl)benzoylimino]-3,4-dimethyl-3H-thiazoline-5-carboxamide hydroiodide (1.0 g), 4,4-ethylenedioxypiperidine (0.5 g), acetic acid (0.2 ml) and methanol (15 ml) was stirred under heating reflux for 1.5 hours. The reaction mixture was concentrated under reduced pressure, and the residue was recrystallized from methylene chloride-toluene to give the title compound (1.0 g).

m.p. 212 - 214°C (decomposed).

Example 4

N-(2-Carboxyethyl)-2-{4-[(4,4-ethylenedioxypiperidin-1-yl)imidoyl]benzoylimino}-3,4-dimethyl-3H-thiazoline-5-carboxamide dihydrate (Compound 5)

A mixture of Compound 4 (0.4 g), 10% aqueous sodium hydroxide solution (1 ml) and methanol (15 ml) was stirred under heating reflux for 40 minutes. The reaction mixture was concentrated under reduced pressure, dissolved in a little amount of water and made neutral with 3 % hydrochloric acid, and the precipitated crystals were collected by filtration to give the title compound (0.3 g).

m.p. 166 - 169°C (decomposed).

**Claims**

1. A thiazoline derivative represented by the formula:

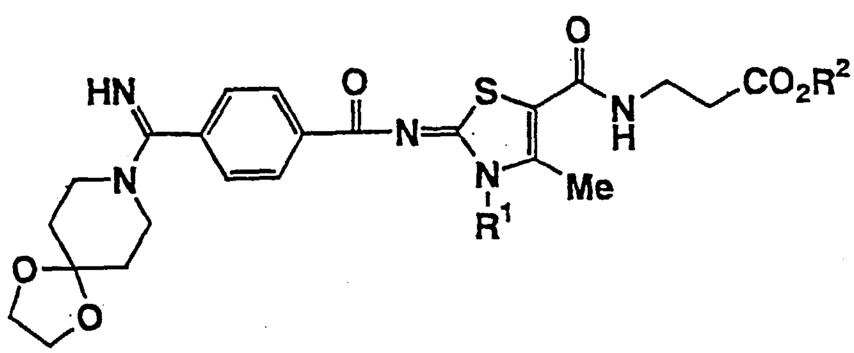

(wherein $R^1$ is an alkyl group having 1 to 4 carbon atoms, and $R^2$ is a hydrogen atom or an alkyl group having 1 to 4 carbon atoms) or a pharmaceutically acceptable salt thereof.

2. A pharmaceutical composition for blood platelet aggregation inhibition comprising the thiazoline derivative or a pharmaceutically acceptable salt according to Claim 1 and a pharmaceutical carrier.

3. Use of the thiazoline derivative or a pharmaceutically acceptable salt according to Claim 1 for blood platelet aggregation inhibition.

## INTERNATIONAL SEARCH REPORT

International application No. PCT/JP95/01361

**A. CLASSIFICATION OF SUBJECT MATTER**

Int. $Cl^6$ C07D491/113, A61K31/435

According to International Patent Classification (IPC) or to both national classification and IPC

**B. FIELDS SEARCHED**

Minimum documentation searched (classification system followed by classification symbols)

Int. $Cl^6$ C07D491/113, A61K31/435

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO, 94-2472, A (Taisho Pharmaceutical Co., Ltd.), February 3, 1994 (03. 02. 94) (Family: none) | 1, 2 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| August 29, 1995 (29. 08. 95) | September 19, 1995 (19. 09. 95) |
| Name and mailing address of the ISA/ Japanese Patent Office | Authorized officer |
| Facsimile No. | Telephone No. |

Form PCT/ISA/210 (second sheet) (July 1992)